# EUROPEAN PATENT APPLICATION

(11) **EP 3 951 006 A1**
(43) Date of publication of application: **09.02.2022**
(21) Application number: 20784999.3
(22) Date of filing: 27.03.2020
(51) Int. Cl.: C23C 16/18, C07C 49/92, C07F 5/00, C23C 16/40, H01L 21/31, H01L 21/316

(54) **RAW MATERIAL FOR FORMING THIN FILM, METHOD FOR PRODUCING THIN FILM, AND SCANDIUM COMPOUND**

(30) Priority: 04.04.2019 JP 2019071820
(71) Applicant: ADEKA CORPORATION, Arakawa-ku Tokyo 116-8554 (JP)
(72) Inventor: YAMADA, Naoki, Tokyo 116-8554 (JP); SATO, Haruyoshi, Tokyo 116-8554 (JP); HARANO, Kazuki, Tokyo 116-8554 (JP)
(74) Representative: Müller-Boré & Partner Patentanwälte PartG mbB
(86) International application number: PCT/JP2020/014047
(87) International publication number: WO 2020/203783

(57) **Abstract**

The present invention provides a thin-film forming raw material including a scandium compound represented by the following general formula (1), a method of producing a thin-film including using the thin-film forming raw material, and a novel scandium compound: 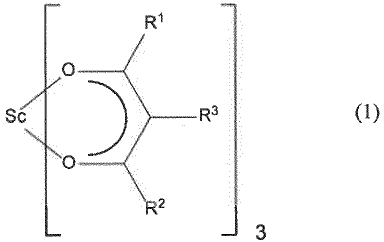 where R¹ represents an alkyl group having 1 to 4 carbon atoms, R² represents an alkyl group having 2 or 3 carbon atoms, and R³ represents a hydrogen atom or an alkyl group having 1 to 4 carbon atoms.

## Description

### Technical Field

The present invention relates to a thin-film forming raw material, a method of producing a thin-film including using the thin-film forming raw material, and a novel compound.

### Background Art

A thin-film forming material including a scandium element shows specific electrical characteristics, and hence has been applied to various technologies. The material has been used as, for example, an electrode material for a memory element typified by a DRAM element, a piezoelectric thin-film, a doping material, and a material for a fuel cell.

As a method of producing the thin-film, there are given, for example, a sputtering method, an ion plating method, MOD methods, such as a coating thermal decomposition method and a sol-gel method, and chemical vapor deposition methods. Of those, the chemical vapor deposition (hereinafter sometimes simply referred to as "CVD") methods including an atomic layer deposition (ALD) method are optimum production processes because the methods each have a number of advantages, such as excellent composition controllability and step coverage, suitability for mass production, and capability of hybrid integration.

Various scandium compounds have heretofore been known as scandium compounds used as thin-film forming raw materials. In each of, for example, Patent Literatures 1 and 2, there is a disclosure of a scandium compound to which diketone groups having specific structures bind. However, the scandium compounds disclosed in Patent Literatures 1 and 2 each have a melting point of 140°C or more, and are hence not compounds sufficiently satisfactory as raw materials, which are used in chemical vapor deposition.

### Citation List

### Patent Literature

[PTL 1] US 2009/0253270 A1
[PTL 2] US 2014/0084355 A1

### Summary of Invention

### Technical Problem

In a method including vaporizing a compound to form a thin-film, such as the CVD method, the compound (precursor) to be used as a raw material is required to satisfy the following conditions: the compound has a low melting point, and hence can be transported under the state of a liquid; the liquid has a low viscosity; the liquid has a large vapor pressure, and hence can be easily vaporized; the compound has high thermal stability; and the compound can produce a high-quality thin-film with high productivity. The compound has been strongly required to satisfy, in particular, the following conditions: the compound has a low melting point, and hence can be transported under the state of a liquid; and the compound can produce a high-quality thin-film with high productivity. However, none of the related-art scandium compounds has been sufficiently satisfactory in terms of those conditions.

Accordingly, an object of the present invention is to provide a thin-film forming raw material including a scandium compound, which can produce a high-quality thin-film with higher productivity than the related-art scandium compounds do, and has a low melting point, and a method of producing a thin-film in which a thin-film containing scandium is formed by using the raw material.

### Solution to Problem

The inventors of the present invention made investigations, and as a result, found that a thin-film forming raw material including a scandium compound having a specific structure, and a method of producing a scandium atom-containing thin-film including using the thin-film forming raw material can solve the above-mentioned problem. Thus, the inventors reached the present invention.

In other words, according to one embodiment of the present invention, there are provided a thin-film forming raw material, comprising a compound represented by the following general formula (1), and a method of producing a thin-film comprising using the raw material: 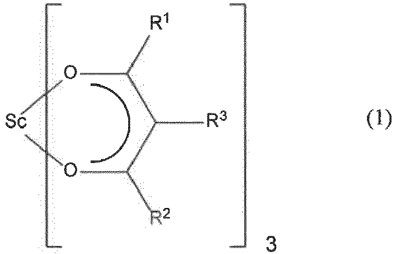 where R¹ represents an alkyl group having 1 to 4 carbon atoms, R² represents an alkyl group having 2 or 3 carbon atoms, and R³ represents a hydrogen atom or an alkyl group having 1 to 4 carbon atoms.

In addition, according to one embodiment of the present invention, there is provided a compound represented by the following general formula (2): 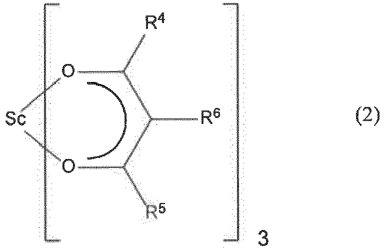 where R⁴ and R⁵ each independently represent an alkyl group having 2 or 3 carbon atoms, and R⁶ represents a hydrogen atom or an alkyl group having 1 to 4 carbon atoms.

### Advantageous Effects of Invention

According to the present invention, the scandium compound having a low melting point can be obtained, and the compound is suitable as a thin-film forming raw material, which is used in a CVD method. In particular, the scandium compound to be used in the present invention has an ALD window, and hence can be suitably used as a thin-film forming raw material, which is used in an ALD method.

### Brief Description of Drawings

FIG. 1 is a schematic diagram for illustrating an example of an apparatus for chemical vapor deposition to be used in a method of producing a thin-film according to the present invention.
FIG. 2 is a schematic diagram for illustrating another example of the apparatus for chemical vapor deposition to be used in the method of producing a thin-film according to the present invention.
FIG. 3 is a schematic diagram for illustrating still another example of the apparatus for chemical vapor deposition to be used in the method of producing a thin-film according to the present invention.
FIG. 4 is a schematic diagram for illustrating yet still another example of the apparatus for chemical vapor deposition to be used in the method of producing a thin-film according to the present invention.

### Description of Embodiments

A thin-film forming raw material of the present invention is a thin-film forming raw material including a compound represented by the general formula (1), is suitable as a precursor for a method of producing a thin-film including a vaporization step, such as a CVD method, and can form a thin-film through use of an ALD method.

In the general formula (1), R¹ represents an alkyl group having 1 to 4 carbon atoms, R² represents an alkyl group having 2 or 3 carbon atoms, and R³ represents a hydrogen atom or an alkyl group having 1 to 4 carbon atoms.

Examples of the alkyl group having 1 to 4 carbon atoms that is represented by each of R¹ and R³ in the general formula (1) include a methyl group, an ethyl group, a n-propyl group, an isopropyl group, a n-butyl group, a sec-butyl group, a tert-butyl group, and an isobutyl group. Examples of the alkyl group having 2 or 3 carbon atoms that is represented by R² therein include an ethyl group, a n-propyl group, and an isopropyl group.

In the general formula (1), R¹, R², and R³ are appropriately selected in accordance with a method of producing a thin-film to which the thin-film forming raw material is applied. When the raw material is used in a method of producing a thin-film including a step of vaporizing the compound, the combination of R¹, R², and R³ is preferably such that the raw material is brought into a liquid state under normal temperature and normal pressure, and has a large vapor pressure.

Specifically, a compound in which R¹ or R² represents an alkyl group having 2 or 3 carbon atoms is preferred, a compound in which R¹ or R² represents an ethyl group is more preferred, and a compound in which both of R¹ and R² represent ethyl groups is particularly preferred because the compounds each have a low melting point. A compound in which R³ represents a hydrogen atom is preferred because the compound has a high vapor pressure. An example of such compound is a compound in which both of R¹ and R² represent ethyl groups, and R³ represents a hydrogen atom (Compound No. 7 below). Meanwhile, in the case of a method of producing a thin-film by a MOD method free of any vaporization step, R¹, R², and R³ in the general formula (1) may be arbitrarily selected in accordance with, for example, solubility in a solvent to be used and a thin-film formation reaction.

Preferred specific examples of the scandium compound represented by the general formula (1) include Compounds No. 1 to No. 30 below.

In the following chemical formulae, "Me" represents a methyl group, "Et" represents an ethyl group, "nPr" represents a n-propyl group, "iPr" represents an isopropyl group, "nBu" represents a n-butyl group, and "tBu" represents a tert-butyl group.

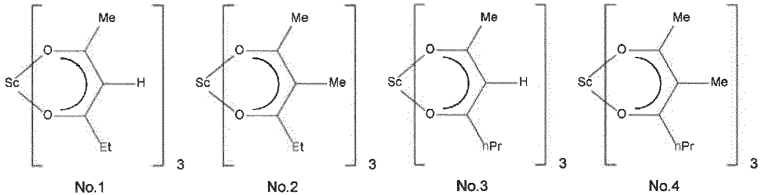

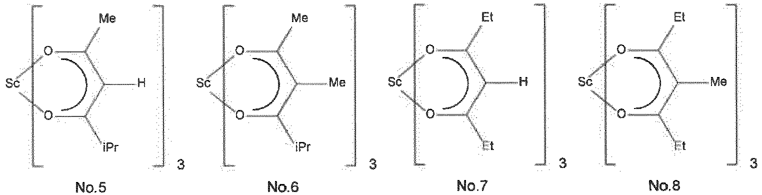

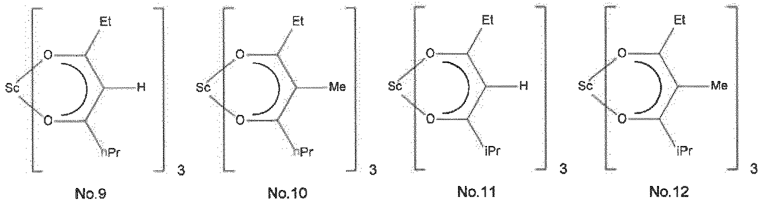

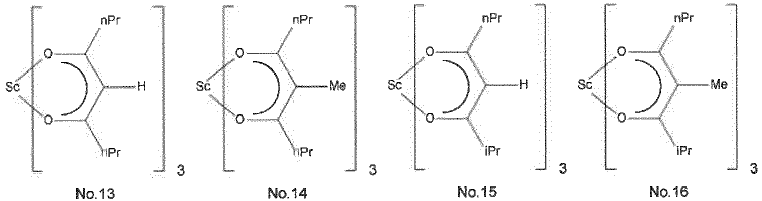

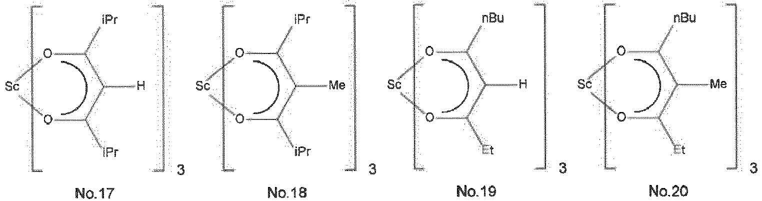

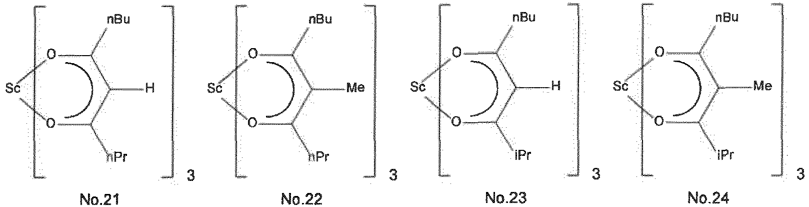

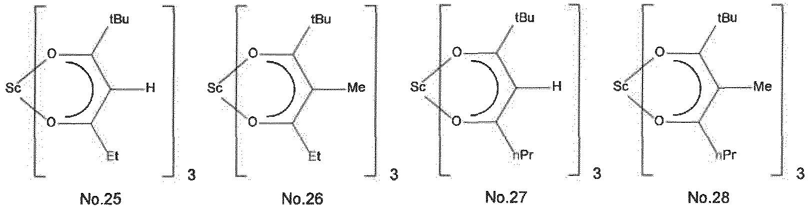

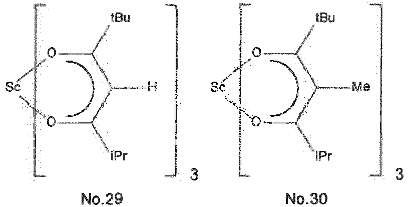

The compound represented by the general formula (1) is not particularly limited by a production method therefor, and is produced by applying a well-known reaction. The compound may be obtained by, for example, a production method including: causing an alkanedione compound having a corresponding structure and an alkanelithium such as butyllithium to react with each other; causing scandium chloride to react with the resultant; and purifying the obtained reaction product through distillation.

Examples of the alkanedione compound include 3,5-heptanedione, 3,5-octanedione, 4,6-nonanedione, 2,6-dimethyl-3,5-heptanedione, and 6-methyl-3,5-heptanedione. Of those, 3,5-heptanedione is preferably used.

Next, the thin-film forming raw material of the present invention includes the above-mentioned scandium compound as a precursor of a thin-film. The form of the thin-film forming raw material varies depending on a production process to which the thin-film forming raw material is applied. For example, when a thin-film containing only scandium as a metal is produced, the thin-film forming raw material of the present invention is free of a metal compound other than the above-mentioned scandium compound and a semimetal compound. Meanwhile, when a thin-film containing two or more kinds of metals and/or a semimetal is produced, the thin-film forming raw material of the present invention may include a compound containing a desired metal and/or a compound containing the semimetal (hereinafter sometimes referred to as "other precursor") in addition to the above-mentioned scandium compound. The thin-film forming raw material of the present invention may further contain an organic solvent and/or a nucleophilic reagent as described later. As described above, the physical properties of the scandium compound serving as a precursor are suitable for a CVD method and an ALD method, and hence the thin-film forming raw material of the present invention is useful, in particular, as a chemical vapor deposition raw material (hereinafter sometimes referred to as "CVD raw material").

When the thin-film forming raw material of the present invention is a chemical vapor deposition raw material, the form thereof is appropriately selected depending on a procedure, such as a transportation and supply method of the CVD method to be used.

As the above-mentioned transportation and supply method, there are given a gas transportation method and a liquid transportation method. The gas transportation method involves heating and/or decompressing the CVD raw material in a container in which the raw material is stored (hereinafter sometimes referred to as "raw material container"), to thereby vaporize the raw material to obtain vapor, and introducing the vapor into a film formation chamber (hereinafter sometimes referred to as "deposition reaction portion") having a substrate set therein together with a carrier gas, such as argon, nitrogen, or helium, to be used as required. The liquid transportation method involves transporting the CVD raw material to a vaporization chamber under a state of a liquid or a solution, heating and/or decompressing the raw material in the vaporization chamber, to thereby vaporize the raw material to obtain vapor, and introducing the vapor into the film formation chamber. In the case of the gas transportation method, the scandium compound represented by the general formula (1) itself may be used as the CVD raw material. In the case of the liquid transportation method, the scandium compound represented by the general formula (1) itself or a solution obtained by dissolving the compound in an organic solvent may be used as the CVD raw material. Those CVD raw materials may further contain the other precursor, a nucleophilic reagent, and the like.

In addition, in a multi-component CVD method, there are given a method involving vaporizing and supplying the CVD raw material independently for each component (hereinafter sometimes referred to as "single source method"), and a method involving vaporizing and supplying a mixed raw material obtained by mixing a multi-component raw material with a desired composition in advance (hereinafter sometimes referred to as "cocktail source method"). In the case of the cocktail source method, a mixture of the scandium compound represented by the general formula (1) and the other precursor or a mixed solution obtained by dissolving the mixture in an organic solvent may be used as the CVD raw material. The mixture or the mixed solution may further contain a nucleophilic reagent and the like.

There is no particular limitation on the above-mentioned organic solvent, and a well-known general organic solvent may be used. Examples of the organic solvent include: acetic acid esters, such as ethyl acetate, butyl acetate, and methoxyethyl acetate; ethers, such as tetrahydrofuran, tetrahydropyran, ethylene glycol dimethyl ether, diethylene glycol dimethyl ether, triethylene glycol dimethyl ether, dibutyl ether, and dioxane; ketones, such as methyl butyl ketone, methyl isobutyl ketone, ethyl butyl ketone, dipropyl ketone, diisobutyl ketone, methyl amyl ketone, cyclohexanone, and methylcyclohexanone; hydrocarbons, such as hexane, cyclohexane, methylcyclohexane, dimethylcyclohexane, ethylcyclohexane, heptane, octane, toluene, and xylene; hydrocarbons each having a cyano group, such as 1-cyanopropane, 1-cyanobutane, 1-cyanohexane, cyanocyclohexane, cyanobenzene, 1,3-dicyanopropane, 1,4-dicyanobutane, 1,6-dicyanohexane, 1,4-dicyanocyclohexane, and 1,4-dicyanobenzene; and pyridine and lutidine. Those organic solvents may be used alone or as a mixed solvent thereof depending on the solubility of a solute, the relationship among the use temperature, the boiling point, and the flash point, and the like. The amount of the entire precursors in the CVD raw material (solution state) obtained by dissolving the precursors in the organic solvent is typically from 0.01 mol/liter to 2.0 mol/liter, preferably from 0.05 mol/liter to 1.0 mol/liter. When the thin-film forming raw material of the present invention is free of a metal compound other than the scandium compound represented by the general formula (1) and a semimetal compound, the amount of the entire precursors refers to the amount of the scandium compound represented by the general formula (1). When the thin-film forming raw material of the present invention includes a compound containing another metal and/or a compound containing a semimetal in addition to the scandium compound, the amount of the entire precursors refers to the total amount of the scandium compound represented by the general formula (1) and the other precursor.

In addition, in the case of the multi-component CVD method, there is no particular limitation on the other precursor to be used together with the scandium compound represented by the general formula (1), and well-known general precursors used in the CVD raw material may be used.

Examples of the other precursor include compounds of one kind or more kinds selected from the group consisting of compounds used as organic ligands, such as an alcohol compound, a glycol compound, a β-diketone compound, a cyclopentadiene compound, and an organic amine compound, and silicon or a metal. In addition, examples of the kind of the metal in the precursor include lithium, sodium, potassium, magnesium, calcium, strontium, barium, titanium, zirconium, hafnium, vanadium, niobium, tantalum, chromium, molybdenum, tungsten, manganese, iron, cobalt, rhodium, iridium, nickel, palladium, platinum, copper, silver, gold, zinc, aluminum, gallium, indium, germanium, tin, lead, antimony, bismuth, yttrium, lanthanum, cerium, praseodymium, neodymium, promethium, samarium, europium, gadolinium, terbium, dysprosium, holmium, erbium, thulium, ytterbium, and lutetium.

Examples of the alcohol compound to be used as the organic ligand in the above-mentioned other precursor include: alkyl alcohols, such as methanol, ethanol, propanol, isopropyl alcohol, butanol, sec-butyl alcohol, isobutyl alcohol, tert-butyl alcohol, pentyl alcohol, isopentyl alcohol, and tert-pentyl alcohol; ether alcohols, such as 2-methoxyethanol, 2-ethoxyethanol, 2-butoxyethanol, 2-(2-methoxyethoxy)ethanol, 2-methoxy-1-methylethanol, 2-methoxy-1,1-dimethylethanol, 2-ethoxy-1,1-dimethylethanol, 2-isopropoxy-1,1-dimethylethanol, 2-butoxy-1,1-dimethylethanol, 2-(2-methoxyethoxy)-1,1-dimethylethanol, 2-propoxy-1,1-diethylethanol, 2-s-butoxy-1,1-diethylethanol, and 3-methoxy-1,1-dimethylpropanol; and dialkylamino alcohols, such as dimethylaminoethanol, ethylmethylaminoethanol, diethylaminoethanol, dimethylamino-2-pentanol, ethylmethylamino-2-pentanol, dimethylamino-2-methyl-2-pentanol, ethylmethylamino-2-methyl-2-pentanol, and diethylamino-2-methyl-2-pentanol.

Examples of the glycol compound to be used as the organic ligand in the above-mentioned other precursor include 1,2-ethanediol, 1,2-propanediol, 1,3-propanediol, 2,4-hexanediol, 2,2-dimethyl-1,3-propanediol, 2,2-diethyl-1,3-propanediol, 1,3-butanediol, 2,4-butanediol, 2,2-diethyl-1,3-butanediol, 2-ethyl-2-butyl-1,3-propanediol, 2,4-pentanediol, 2-methyl-1,3-propanediol, 2-methyl-2,4-pentanediol, 2,4-hexanediol, and 2,4-dimethyl-2,4-pentanediol.

In addition, examples of the β-diketone compound include: alkyl-substituted β-diketones, such as acetylacetone, hexane-2,4-dione, 5-methylhexane-2,4-dione, heptane-2,4-dione, 2-methylheptane-3,5-dione, 5-methylheptane-2,4-dione, 6-methylheptane-2,4-dione, 2,2-dimethylheptane-3,5-dione, 2,6-dimethylheptane-3,5-dione, 2,2,6-trimethylheptane-3,5-dione, 2,2,6,6-tetramethylheptane-3,5-dione, octane-2,4-dione, 2,2,6-trimethyloctane-3,5-dione, 2,6-dimethyloctane-3,5-dione, 2,9-dimethylnonane-4,6-dione, 2-methyl-6-ethyldecane-3,5-dione, and 2,2-dimethyl-6-ethyldecane-3,5-dione; fluorine-substituted alkyl β-diketones, such as 1,1,1-trifluoropentane-2,4-dione, 1,1,1-trifluoro-5,5-dimethylhexane-2,4-dione, 1,1,1,5,5,5-hexafluoropentane-2,4-dione, and 1,3-diperfluorohexylpropane-1,3-dione; and ether-substituted β-diketones, such as 1,1,5,5-tetramethyl-1-methoxyhexane-2,4-dione, 2,2,6,6-tetramethyl-1-methoxyheptane-3,5-dione, and 2,2,6,6-tetramethyl-1-(2-methoxyethoxy)heptane-3,5-dione.

In addition, examples of the cyclopentadiene compound include cyclopentadiene, methylcyclopentadiene, ethylcyclopentadiene, propylcyclopentadiene, isopropylcyclopentadiene, butylcyclopentadiene, sec-butylcyclopentadiene, isobutylcyclopentadiene, tert-butylcyclopentadiene, dimethylcyclopentadiene, and tetramethylcyclopentadiene, and examples of the organic amine compound to be used as the above-mentioned organic ligand include methylamine, ethylamine, propylamine, isopropylamine, butylamine, sec-butylamine, tert-butylamine, isobutylamine, dimethylamine, diethylamine, dipropylamine, diisopropylamine, ethylmethylamine, propylmethylamine, and isopropylmethylamine.

The above-mentioned other precursors are known in the art, and production methods therefor are also known. One example of the production methods is given as described below. For example, when the alcohol compound is used as the organic ligand, the precursor may be produced through a reaction between an inorganic salt of the metal described above or a hydrate thereof and an alkali metal alkoxide of the alcohol compound. In this case, examples of the inorganic salt of the metal or the hydrate thereof may include a halide and a nitrate of the metal, and examples of the alkali metal alkoxide may include a sodium alkoxide, a lithium alkoxide, and a potassium alkoxide.

In the case of the single source method, a compound similar to the scandium compound represented by the general formula (1) in the behavior of thermal decomposition and/or oxidative decomposition is preferably used as the above-mentioned other precursor. In the case of the cocktail source method, a compound that not only is similar to the scandium compound represented by the general formula (1) in the behavior of thermal decomposition and/or oxidative decomposition but also does not cause any change impairing desired characteristics as a precursor through a chemical reaction or the like at the time of mixing is preferably used as the above-mentioned other precursor.

In addition, the thin-film forming raw material of the present invention may contain a nucleophilic reagent as required in order to impart stability to the scandium compound represented by the general formula (1) and the other precursor. Examples of the nucleophilic reagent include: ethylene glycol ethers, such as glyme, diglyme, triglyme, and tetraglyme; crown ethers, such as 18-crown-6, dicyclohexyl-18-crown-6, 24-crown-8, dicyclohexyl-24-crown-8, and dibenzo-24-crown-8; polyamines, such as ethylenediamine, N,N'-tetramethylethylenediamine, diethylenetriamine, triethylenetetramine, tetraethylenepentamine, pentaethylenehexamine, 1,1,4,7,7-pentamethyldiethylenetriamine, 1,1,4,7,10,10-hexamethyltriethylenetetramine, and triethoxytriethyleneamine; cyclic polyamines, such as cyclam and cyclen; heterocyclic compounds, such as pyridine, pyrrolidine, piperidine, morpholine, N-methylpyrrolidine, N-methylpiperidine, N-methylmorpholine, tetrahydrofuran, tetrahydropyran, 1,4-dioxane, oxazole, thiazole, and oxathiolane; β-keto esters, such as methyl acetoacetate, ethyl acetoacetate, and 2-methoxyethyl acetoacetate; and β-diketones, such as acetylacetone, 2,4-hexanedione, 2,4-heptanedione, 3,5-heptanedione, and dipivaloylmethane. The usage amount of each of those nucleophilic reagents falls within the range of preferably from 0.1 mol to 10 mol, more preferably from 1 mol to 4 mol with respect to 1 mol of the amount of the entire precursors.

The thin-film forming raw material of the present invention is prevented from containing impurity metal elements other than the components forming the raw material, impurity halogens, such as impurity chlorine, and impurity organic substances to the extent possible. The content of each of the impurity metal elements is preferably 100 ppb or less, more preferably 10 ppb or less, and the total content thereof is preferably 1 ppm or less, more preferably 100 ppb or less. In particular, when the raw material is used as a gate insulating film, a gate film, or a barrier layer of an LSI, it is required to reduce the contents of alkali metal elements and alkaline-earth metal elements that influence the electrical characteristics of a thin-film to be obtained. The content of the impurity halogens is preferably 100 ppm or less, more preferably 10 ppm or less, most preferably 1 ppm or less. The total content of the impurity organic substances is preferably 500 ppm or less, more preferably 50 ppm or less, most preferably 10 ppm or less. In addition, moisture causes generation of particles in the chemical vapor deposition raw material and generation of particles during thin-film formation. Accordingly, moisture in each of the precursor, the organic solvent, and the nucleophilic reagent is preferably removed as much as possible before its use. The moisture content of each of the precursor, the organic solvent, and the nucleophilic reagent is preferably 10 ppm or less, more preferably 1 ppm or less.

In addition, it is preferred that the thin-film forming raw material of the present invention be prevented from containing particles to the extent possible in order to reduce or prevent particle contamination of a thin-film to be formed. Specifically, in particle measurement with a light scattering liquid particle detector in a liquid phase, it is preferred that the number of particles larger than 0.3 µm be 100 or less in 1 mL of the liquid phase, it is more preferred that the number of particles larger than 0.2 µm be 1,000 or less in 1 mL of the liquid phase, and it is most preferred that the number of particles larger than 0.2 µm be 100 or less in 1 mL of the liquid phase.

A method of producing a thin-film of the present invention in which the thin-film is produced by using the thin-film forming raw material of the present invention is a CVD method including: introducing vapor obtained by vaporizing the thin-film forming raw material of the present invention and a reactive gas to be used as required into a film formation chamber (treatment atmosphere) having a substrate set therein; and then subjecting the precursor to decomposition and/or a chemical reaction on the substrate, to thereby grow and deposit the thin-film containing a metal on the surface of the substrate. There are no particular limitations on a transportation and supply method for the raw material, a deposition method therefor, production conditions, a production apparatus, and the like, and well-known general conditions and methods may be used.

Examples of the above-mentioned reactive gas to be used as required include: oxidizing gases, such as oxygen, ozone, nitrogen dioxide, nitrogen monoxide, water vapor, hydrogen peroxide, formic acid, acetic acid, and acetic anhydride; reducing gases, such as hydrogen; and gases to produce nitrides, such as organic amine compounds including a monoalkylamine, a dialkylamine, a trialkylamine, and an alkylenediamine, hydrazine, and ammonia. Those gases may be used alone or in combination thereof. The thin-film forming raw material of the present invention has satisfactory reactivity with ozone out of those gases. Accordingly, when one kind is used as the reactive gas, ozone is preferably used, and when a mixed gas of two or more kinds is used as the reactive gas, the mixed gas preferably contains at least ozone.

In addition, examples of the above-mentioned transportation and supply method include the gas transportation method, the liquid transportation method, the single source method, and the cocktail source method described above.

In addition, examples of the above-mentioned deposition method include: thermal CVD including causing a raw material gas, or the raw material gas and a reactive gas, to react only with heat, to thereby deposit a thin-film; plasma CVD using heat and plasma; optical CVD using heat and light; optical plasma CVD using heat, light, and plasma; and ALD including dividing a deposition reaction of CVD into elementary steps, and performing deposition at a molecular level in a stepwise manner.

As a material for the substrate, there are given, for example: silicon; ceramics, such as silicon nitride, titanium nitride, tantalum nitride, titanium oxide, titanium nitride, scandium oxide, zirconium oxide, hafnium oxide, and lanthanum oxide; glass; and metals, such as metal cobalt. The shape of the substrate is, for example, a plate shape, a spherical shape, a fibrous shape, or a scaly shape. The surface of the substrate may be planar, or may have a three-dimensional structure, such as a trench structure.

In addition, examples of the above-mentioned production conditions include a reaction temperature (substrate temperature), a reaction pressure, and a deposition rate. The reaction temperature is preferably not less than 100°C that is the temperature at which the compound of the present invention sufficiently reacts, more preferably from 150°C to 400°C, particularly preferably from 200°C to 350°C. In addition, the reaction pressure is preferably from 10 Pa to an atmospheric pressure in the case of the thermal CVD or the optical CVD, and is preferably from 10 Pa to 2,000 Pa in the case of using plasma.

In addition, the deposition rate may be controlled by the supply conditions (vaporization temperature and vaporization pressure) of the raw material, the reaction temperature, and the reaction pressure. When the deposition rate is large, the characteristics of a thin-film to be obtained may deteriorate. When the deposition rate is small, a problem may occur in productivity. Accordingly, the deposition rate is preferably from 0.01 nm/min to 100 nm/min, more preferably from 1 nm/min to 50 nm/min. In addition, in the case of the ALD method, the deposition rate is controlled by the number of cycles so that a desired film thickness may be obtained.

As the above-mentioned production conditions, there are further given a temperature and a pressure when the thin-film forming raw material is vaporized to obtain vapor. The step of vaporizing the thin-film forming raw material to obtain vapor may be performed in the raw material container or in the vaporization chamber. In any case, it is preferred that the thin-film forming raw material of the present invention be evaporated at a temperature of from 0°C to 150°C. In addition, when the thin-film forming raw material is vaporized to obtain vapor in the raw material container or in the vaporization chamber, the pressure in the raw material container and the pressure in the vaporization chamber are both preferably from 1 Pa to 10,000 Pa.

When the ALD method is adopted, the method of producing a thin-film of the present invention may include, in addition to a raw material introduction step of vaporizing the thin-film forming raw material by the above-mentioned transportation and supply method to provide vapor, followed by the introduction of the vapor into the film formation chamber, a precursor thin-film formation step of forming a precursor thin-film from the above-mentioned compound in the vapor on the surface of the above-mentioned substrate, an evacuation step of evacuating an unreacted compound gas, and a metal-containing thin-film formation step of causing the precursor thin-film to chemically react with the reactive gas, to thereby form a thin-film containing a metal on the surface of the substrate.

Now, regarding each step of the ALD method, the case of forming a metal oxide thin-film is described in detail as an example. First, the above-mentioned raw material introduction step is performed. The preferred temperature and pressure when the thin-film forming raw material is turned into vapor are the same as the conditions described in the method of producing a thin-film by the CVD method. Next, the vapor introduced into the film formation chamber and the surface of the substrate are brought into contact with each other, and hence the precursor thin-film is formed on the surface of the substrate (precursor thin-film formation step). In this case, heat may be applied by heating the substrate or heating the film formation chamber. The precursor thin-film formed in this step is a thin-film produced from the compound represented by the general formula (1) or a thin-film produced by the decomposition and/or reaction of part of the compound represented by the general formula (1), and hence has composition different from that of the target metal oxide thin-film. The temperature of the substrate when this step is performed is preferably from room temperature to 500°C, more preferably from 150°C to 350°C. The pressure of a system (in the film formation chamber) when this step is performed is preferably from 1 Pa to 10,000 Pa, more preferably from 10 Pa to 1,000 Pa.

Next, the unreacted compound gas and a gas generated as a by-product are evacuated from the film formation chamber (evacuation step). It is ideal that the unreacted compound gas and the gas generated as a by-product be completely evacuated from the film formation chamber, but it is not always required that the gases be completely evacuated. Examples of an evacuation method include: a method including purging the inside of the system with an inert gas, such as nitrogen, helium, or argon; a method including performing evacuation by decompressing the inside of the system; and a combination of these methods. When the decompression is performed, the pressure in the system is set to preferably from 0.01 Pa to 300 Pa, more preferably from 0.01 Pa to 100 Pa.

Next, an oxidizing gas is introduced as the reactive gas into the film formation chamber, and the metal oxide thin-film is formed from the precursor thin-film obtained in the previous precursor thin-film formation step through the action of the oxidizing gas or the action of the oxidizing gas and heat (metal oxide-containing thin-film formation step). In this step, the temperature when the heat is applied is preferably from room temperature to 500°C, more preferably from 150°C to 350°C. The pressure of the system (in the film formation chamber) when this step is performed is preferably from 1 Pa to 10,000 Pa, more preferably from 10 Pa to 1,000 Pa. The compound of the present invention has satisfactory reactivity with the oxidizing gas, and hence a high-quality metal oxide thin-film containing less residual carbon can be obtained.

When the ALD method is adopted in the method of producing a thin-film of the present invention as described above, thin-film deposition performed by a series of operations including the above-mentioned raw material introduction step, precursor thin-film formation step, evacuation step, and metal oxide-containing thin-film formation step is defined as one cycle, and this cycle may be repeated a plurality of times until a thin-film having a required film thickness is obtained. In this case, it is preferred that, after one cycle is performed, a compound gas and a reactive gas (oxidizing gas when the metal oxide thin-film is formed) that are unreacted, and a gas generated as a by-product be evacuated from the deposition reaction portion in the same manner as in the above-mentioned evacuation step, and then the subsequent one cycle be performed.

In addition, in the formation of the metal oxide thin-film by the ALD method, energy such as plasma, light, or a voltage may be applied, and a catalyst may be used. There are no particular limitations on the timing for applying the energy and the timing for using the catalyst. The energy may be applied or the catalyst may be used, for example, at the time of introducing the compound gas in the raw material introduction step, at the time of heating in the precursor thin-film formation step or the metal oxide-containing thin-film formation step, at the time of evacuating the inside of the system in the evacuation step, or at the time of introducing the oxidizing gas in the metal oxide-containing thin-film formation step, or between the above-mentioned respective steps.

In addition, in the method of producing a thin-film of the present invention, after the thin-film deposition, annealing treatment may be performed in an inert atmosphere, an oxidizing atmosphere, or a reducing atmosphere in order to obtain more satisfactory electrical characteristics. When step embedding is required, a reflow step may be provided. The temperature in this case is typically from 200°C to 1,000°C, preferably from 250°C to 500°C.

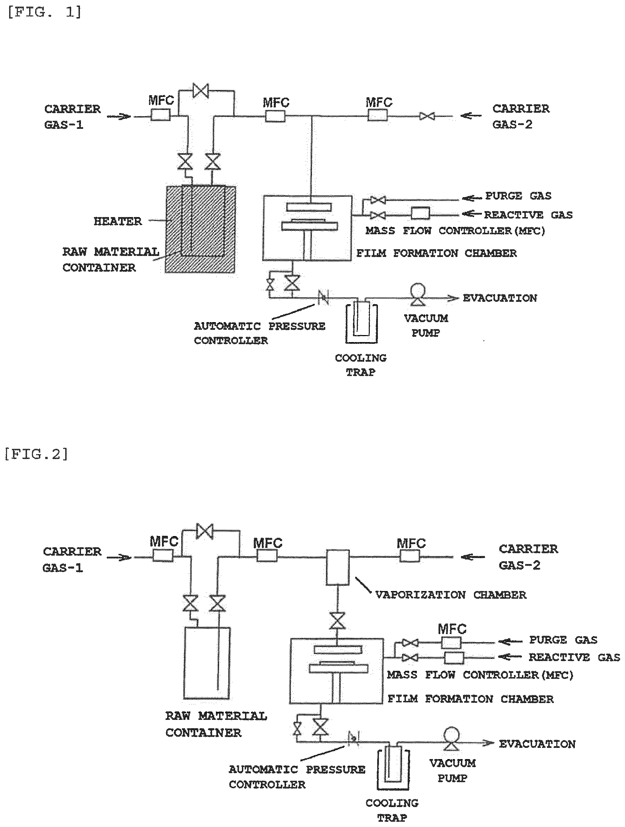

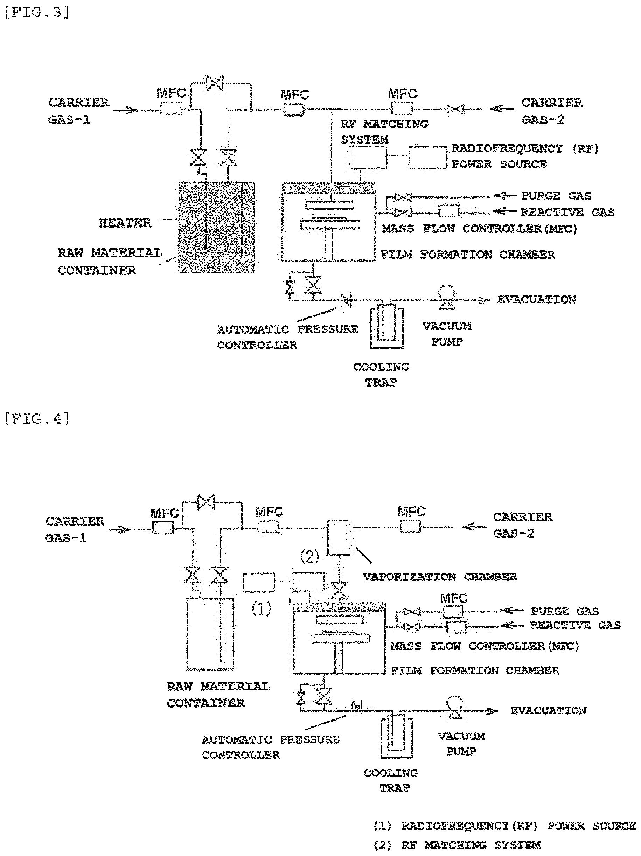

As an apparatus for producing a thin-film through use of the thin-film forming raw material of the present invention, a well-known apparatus for a chemical vapor deposition method may be used. As specific examples of the apparatus, there are given an apparatus capable of performing bubbling supply of a precursor as illustrated in FIG. 1 and an apparatus including a vaporization chamber as illustrated in FIG. 2. In addition, there is given an apparatus capable of subjecting the reactive gas to plasma treatment as illustrated in FIG. 3 and FIG. 4. The apparatus is not limited to single-substrate type apparatus as illustrated in FIG. 1 to FIG. 4, and an apparatus capable of simultaneously processing a large number of substrates through use of a batch furnace may also be used.

A thin-film produced through use of the thin-film forming raw material of the present invention may be formed as desired kinds of thin-films, such as thin-films of a metal, oxide ceramics, nitride ceramics, and glass, by appropriately selecting the other precursor, the reactive gas, and the production conditions. It has been known that the thin-films exhibit electrical characteristics, optical characteristics, and the like. Examples thereof include a metal scandium thin-film, a scandium oxide thin-film, a scandium alloy, and a scandium-containing composite oxide thin-film. An example of the scandium alloy is an Al-Sc alloy. Those thin-films are applied to various products, and have been widely used in the production of, for example, electrode materials for memory elements typified by DRAM elements, resistance films, diamagnetic films used for the recording layers of hard disks, and catalyst materials for polymer electrolyte fuel cells.

Of the above-mentioned thin-film forming raw materials, a compound represented by the general formula (2) is also novel as a compound. The novel compound of the present invention is a compound, which has a low melting point, can be adapted to the ALD method, and is particularly suitable as a precursor for a method of producing a thin-film including a vaporization step, such as the CVD method.

In the general formula (2), R⁴ and R⁵ each independently represent an alkyl group having 2 or 3 carbon atoms, and R⁶ represents a hydrogen atom or an alkyl group having 1 to 4 carbon atoms. Examples of the alkyl group having 2 or 3 carbon atoms include an ethyl group, a n-propyl group, and an isopropyl group.

A case in which R⁴ or R⁵ in the general formula (2) represents an ethyl group is preferred because a low melting point is obtained, and a case in which both of R⁴ and R⁵ represent ethyl groups is particularly preferred. R⁶ therein preferably represents a hydrogen atom because the compound has a high vapor pressure. An example of such compound is a compound in which both of R⁴ and R⁵ represent ethyl groups, and R⁶ represents a hydrogen atom (Compound No. 7 above).

The compound represented by the general formula (2) is not particularly limited by a production method therefor, and is produced by applying a well-known reaction. As in the method of producing the compound represented by the general formula (1), the compound may be obtained by, for example, a production method including: causing scandium chloride and an alkanedione compound having a corresponding structure to react with each other; and purifying the resultant through distillation.

Specific examples of the novel compound represented by the general formula (2) include compounds represented by Compounds No. 7 to No. 18 above.

### Examples

The present invention is described in more detail below by way of the Examples, the Production Example, the Comparative Example, and the Evaluation Examples. However, the present invention is by no means limited by Examples and the like below.

### [Example 1] Synthesis of Compound No. 7

2.1 g (1.6×10⁻² mol) of 3,5-heptanedione and 16 g (0.22 mol) of dehydrated tetrahydrofuran were added to a 100-milliliter four-necked flask, and the mixture was cooled to - 40°C. After that, 9.9 ml (1.6 mol/l) of a n-butyllithium/hexane solution was added dropwise thereto. The temperature of the mixture was increased to room temperature, and the mixture was stirred for 6 hours. The mixture was added dropwise to a mixed liquid of 0.80 g (5.3×10⁻³ mol) of anhydrous ScCl₃ and 20 g (0.22 mol) of toluene. 10 g (0.11 mol) of toluene was added to the reaction liquid, and the mixture was stirred under reflux in an oil bath at 110°C for 12 hours. Under reduced pressure, the solvent was removed from the mixture in an oil bath at 80°C, and 20 g (0.23 mol) of hexane was added to the residue, followed by filtration. Under reduced pressure, the solvent was removed from the filtrate in an oil bath at 70°C. The obtained orange liquid was purified by distillation at a heating temperature of 160°C and a pressure of 30 Pa. Thus, 1.1 g of a colorless and transparent liquid was obtained.

### (Analytic Values)

### (1) Normal-pressure TG-DTA

50% mass loss temperature: 253°C (Ar flow rate: 100 ml/min, temperature increase rate: 10°C/min)

### (2) Reduced-pressure TG-DTA

50% mass loss temperature: 174°C (Ar flow rate: 50 ml/min, temperature increase rate: 10°C/min)

### (3) ¹H-NMR (Deuterated Benzene)

1.01 ppm (6H, triplet), 2.05 ppm (4H, quartet), 5.43 ppm (1H, singlet)

### (4) Elemental Analysis (Theoretical Values)

Sc: 10.7% (10.56%), C: 59.3% (59.15%), H: 7.6% (7.75%), O: 22.4% (22.54%)

### [Evaluation Example 1, and Comparative Evaluation Examples 1 and 2] Evaluation of Physical Properties of Scandium Compounds

The states of Compound No. 7 of the present invention obtained in Example 1, and Comparative Compounds 1 and 2 below at 25°C were visually observed. Compounds that were solids at 25°C were measured for their melting points. The results thereof are shown in Table 1. In the chemical formulae of Comparative Compound 1 and Comparative Compound 2 below, "Me" represents a methyl group and "tBu" represents a tert-butyl group.

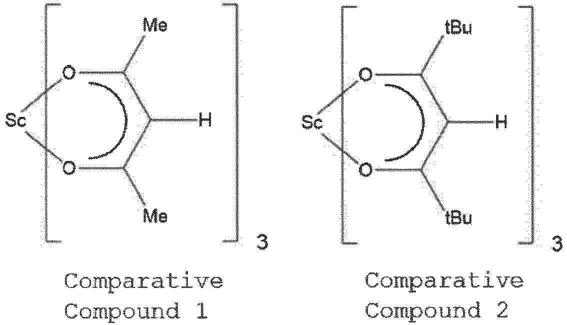

**Table 1**

| | Compound | State at 25°C | Melting point/°C |
|---|---|---|---|
| Evaluation Example 1 | No. 7 | Liquid | - |
| Comparative Evaluation Example 1 | Comparative Compound 1 | Solid | 185 |
| Comparative Evaluation Example 2 | Comparative Compound 2 | Solid | 150 |

It was found from the results of Table 1 that Compound No. 7 had a melting point much lower than those of Comparative Compound 1 and Comparative Compound 2.

### [Example 2] Production of Scandium Oxide Thin-film

A scandium oxide thin-film was produced on a silicon wafer by the ALD method under the following conditions through use of an apparatus illustrated in FIG. 1 with Compound No. 7 being used as a raw material, which was used in an atomic layer deposition method.

When the composition of the obtained thin-film was checked by X-ray photoelectron spectroscopy, the obtained thin-film was scandium oxide, and its residual carbon content was less than 1.0 atom%. In addition, when its film thickness was measured by an X-ray reflectivity method, and the average value thereof was calculated, the average film thickness was 25.7 nm, and the average film thickness obtained per cycle was 0.05 nm.

### (Conditions)

Substrate: silicon wafer
Reaction temperature (silicon wafer temperature): 300°C
Reactive gas: ozone

A series of steps including the following (1) to (4) was defined as one cycle, and this cycle was repeated 500 times:
(1) a raw material, which is used in an atomic layer deposition method, vaporized under the conditions of a raw material container temperature of 120°C and a raw material container internal pressure of 100 Pa is introduced into a film formation chamber and deposited at a system pressure of 100 Pa for 30 seconds;
(2) the raw material which has not been deposited is removed through argon purging for 15 seconds;
(3) a reactive gas is introduced into the film formation chamber and subjected to a reaction at a system pressure of 100 Pa for 5 second; and
(4) an unreacted reactive gas and a by-product gas are removed through argon purging for 15 seconds.

### [Example 3] Production of Scandium Oxide Thin-film

A scandium oxide thin-film was produced under the same conditions as those in Example 2 except that Compound No. 1 was used as a raw material, which was used in an atomic layer deposition method. When the composition of the obtained thin-film was checked by X-ray photoelectron spectroscopy, the obtained thin-film was scandium oxide, and its residual carbon content was less than 1.0 atom%. In addition, when its film thickness was measured by an X-ray reflectivity method, and the average value thereof was calculated, the average film thickness was 15.5 nm, and the average film thickness obtained per cycle was 0.03 nm.

### [Comparative Example 1] Production of Scandium Oxide Thin-film

A scandium oxide thin-film was produced under the same conditions as those in Example 2 except that Comparative Compound 1 was used as a raw material, which was used in an atomic layer deposition method. When the composition of the obtained thin-film was checked by X-ray photoelectron spectroscopy, the obtained thin-film was scandium oxide, and its residual carbon content was 5.0 atom%. In addition, when its film thickness was measured by an X-ray reflectivity method, and the average value thereof was calculated, the average film thickness was 5.1 nm, and the average film thickness obtained per cycle was 0.01 nm.

It was found from the results in Examples 2 and 3 that, in each of the Examples, a high-quality scandium oxide thin-film having a low residual carbon content was able to be produced. Meanwhile, it was found that the thin-film obtained in Comparative Example 1 was a low-quality scandium oxide thin-film having an extremely high residual carbon content. In addition, comparison among the film thicknesses obtained per cycle in Examples 2 and 3, and Comparative Example 1 showed that, in each of Examples 2 and 3, the scandium oxide thin-film was able to be produced with productivity three or more times as high as that of Comparative Example 1. In particular, it was found that, in Example 2, the scandium oxide thin-film was able to be produced with extremely high productivity.

It was found from the foregoing results that according to the present invention, a high-quality scandium oxide thin-film was able to be produced by the ALD method with high productivity.

## Claims

1. A thin-film forming raw material, comprising a scandium compound represented by the following general formula (1): 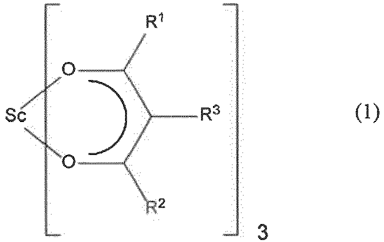 where R¹ represents an alkyl group having 1 to 4 carbon atoms, R² represents an alkyl group having 2 or 3 carbon atoms, and R³ represents a hydrogen atom or an alkyl group having 1 to 4 carbon atoms.

2. A method of producing a thin-film containing a scandium atom on a surface of a substrate, comprising the steps of:
vaporizing the thin-film forming raw material of claim 1;
introducing vapor containing the vaporized scandium compound represented by the general formula (1) into a treatment atmosphere; and
subjecting the compound to decomposition and/or a chemical reaction, to thereby deposit the compound on the surface of the substrate.

3. A use of a scandium compound represented by the following general formula (1) for producing a thin-film containing a scandium atom on a surface of a substrate: 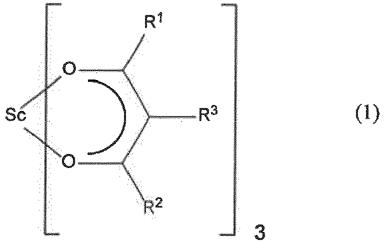 where R¹ represents an alkyl group having 1 to 4 carbon atoms, R² represents an alkyl group having 2 or 3 carbon atoms, and R³ represents a hydrogen atom or an alkyl group having 1 to 4 carbon atoms.

4. A scandium compound represented by the following general formula (2): 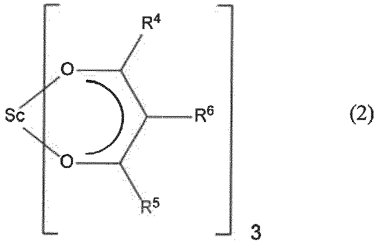 where R⁴ and R⁵ each independently represent an alkyl group having 2 or 3 carbon atoms, and R⁶ represents a hydrogen atom or an alkyl group having 1 to 4 carbon atoms.
